# EUROPEAN PATENT APPLICATION

(11) **EP 4 552 552 A1**
(43) Date of publication of application: **14.05.2025**
(21) Application number: 23835849.3
(22) Date of filing: 06.07.2023
(51) Int. Cl.: A61B 5/00, G16H 10/20, G16H 50/50

(54) **COGNITIVE ABILITY TESTING SYSTEM**

(30) Priority: 06.07.2022 KR 20220083335
(71) Applicant: Peoplebio Inc., Seongnam-si Gyeonggi-do 13487 (KR)
(72) Inventor: KIM, Sangyun, Seoul 06574 (KR)
(74) Representative: J A Kemp LLP
(86) International application number: PCT/KR2023/009543
(87) International publication number: WO 2024/010383

(57) **Abstract**

The present invention relates to a method and a system for testing cognitive ability. By using the method and testing system of the present invention, a highly effective and reliable test related to frontal lobe activity can be performed. The test results can be utilized to provide information for the diagnosis of diseases that lead to cognitive impairment.

## Description

### Technical Field

This application is based on and claims priority from Korean Patent Application No. 10-2022-0083335, filed on July 06, 2022, with the Korean Intellectual Property Office, the disclosure of which is incorporated herein by reference in its entirety.

The disclosure relates to a method for testing cognitive ability and a testing system using the same.

### Background Art

The frontal lobe is anatomically divided into the primary motor area adjacent to the central sulcus, the premotor area located in front thereof, and the prefrontal area collectively indicating the remaining areas. The primary motor area and the premotor area are involved in motor performance and action output functions. On the other hand, the prefrontal area sets behavioral goals and performs a management function of mobilizing, coordinating, and executing the functions of other brain areas in order to attain the goals. The prefrontal area has neural connections with the cerebral cortex areas that provide sensory information and receives input from subcortical structures such as the limbic system and pituitary gland that provide information about the internal state of the body, thereby having a neural connection structure capable of collecting and synthesizing various information and coordinating the functions of other brain areas. The Trail Making Test (TMT) is a neuropsychological test widely used to evaluate cognitive functions, especially the frontal lobe and executive functions. The TMT is one of the most sensitive tests for identifying mild cognitive impairment and mild dementia. The TMT is configured by Parts A and B, which assess various cognitive processes. The TMT Part A requires participants to connect randomly distributed numbers in ascending order, while the TMT Part B includes both numbers and letters and requires participants to connect numbers and letters alternately. The TMT Part A primarily addresses attention, while the TMT Part B is used as an index of frontal lobe executive function.

The existing TMT exhibits limited utilization because it uses the English alphabet in the TMT Part B. This has led to a bias toward low performance on the TMT-B among non-English speakers and illiterate older adults. Many previous studies have reported that older adults with low levels of education and patients with mild cognitive impairment in non-English speaking countries often perform poorly on the TMT Part B. To overcome these shortcomings, several versions of TMT have been developed for illiterate and non-native English speakers. The Color Trail Test (CTT) uses two colors, and the Shape Trail Test (STT) uses circles and squares. Although the validity and reliability of the CTT have been established, the CTT has not been considered comparable to the TMT because of the likelihood of the Stroop effect on the CTT-B. Furthermore, the CTT is difficult to apply to people with color blindness or visual impairment. There have been efforts in several non-English speaking countries to develop TMTs using their own language. However, these tests still require validation and have very limited utility.

### Disclosure of Invention

### Technical Problem

The disclosure has been made to solve the problems described above, and the inventors have made a great effort to develop a method for performing a non-invasive cognitive ability test related to frontal lobe activation easily and reliably within a short period of time. As a result, the inventors identified that a highly reliable cognitive ability test is possible by having the assessment subjects perform a number arrangement according to a predetermined rule and by arranging intruders according to a predetermined rule during the above arrangement process, thereby having completed the disclosure

Therefore, the disclosure is to provide a cognitive function assessment test board.

Also, the disclosure is to provide a method for providing information for cognitive function assessment.

Also, the disclosure is to provide a device for providing a cognitive function test.

Also, the disclosure is to provide a computer program for cognitive function assessment.

### Solution to Problem

In view of the foregoing, according to one aspect of the disclosure, there is provided a cognitive function assessment test board on which numbers n to n+i (n is an integer greater than or equal to 0 and i is an integer greater than or equal to 3) are arranged, wherein n is displayed once, n+1 to n+i are displayed twice, respectively, and two identical numbers displayed twice are displayed by a first notation method and a second notation method, which are distinct from each other, respectively, wherein the numbers are classified into two groups of couplers and intruders, wherein the couplers include n defined as a starting point and the n is displayed by the first notation method or the second notation method, wherein the couplers include n+b (b is an odd number greater than or equal to 1 and less than or equal to i) displayed by a notation method different from n, among the first notation method and the second notation method, and n+a (a is an even number greater than or equal to 2 and less than or equal to i) displayed by the same notation method as n, and wherein the intruders include n+a (a is an even number greater than or equal to 2 and less than or equal to i) displayed by a notation method different from n, among the first notation method and the second notation method, and n+b (b is an odd number greater than or equal to 1 and less than or equal to i) displayed by the same notation method as n.

According to an embodiment, in the test board, when a distance between n+x and n+x+1 (x is an integer greater than or equal to 0 and less than or equal to i-1) belonging to the couplers is defined as D1, a distance between n+x belonging to the couplers and n+x+1 belonging to the intruders is defined as D2, and a distance between n+x+1 belonging to the intruders and n+x+1 belonging to the couplers is defined as D3, conditions D1≥D2 and D1≥D3 are satisfied.

According to another embodiment, the first notation method and the second notation method may be different from each other in one or more selected from a group including a font color of the displayed number, a shade color of the displayed number, and a shade shape of the displayed number.

According to another embodiment, the first notation method and the second notation method may be different from each other in the shade colors of the displayed numbers.

According to another embodiment, the first notation method and the second notation method may be distinguished from each other by the numbers being displayed differently in black and white, respectively, as the shade colors of the numbers.

According to another embodiment, the D1 is greater than the D2 and the D3.

According to another embodiment, the i may be an integer of 2 to 49.

According to another embodiment, the i may be an integer of 9 to 29.

According to another embodiment, when connecting the numbers belonging to the couplers in the order from the smallest to the biggest using line segments, none of the line segments intersect each other.

According to another aspect of the disclosure, there is provided a method of providing information for cognitive function assessment, which includes: randomly displaying numbers n to n+i (n is an integer greater than or equal to 0 and i is an integer greater than or equal to 3) on a screen; receiving information about numbers clicked or sequentially connected by a user; and determining whether the numbers belonging to couplers are sequentially clicked or sequentially connected from the smallest to the biggest, based on the information received from the user, and displaying a result thereof on the screen, wherein n is displayed once, n+1 to n+i are displayed twice, respectively, and two identical numbers displayed twice are displayed by a first notation method and a second notation method, which are distinct from each other, respectively, wherein the numbers are classified into two groups of couplers and intruders, wherein the couplers include n defined as a starting point and the n is displayed by the first notation method or the second notation method, wherein the couplers include n+b (b is an odd number greater than or equal to 1 and less than or equal to i) displayed by a notation method different from n, among the first notation method and the second notation method, and n+a (a is an even number greater than or equal to 2 and less than or equal to i) displayed by the same notation method as n, and wherein the intruders include n+a (a is an even number greater than or equal to 2 and less than or equal to i) displayed by a notation method different from n, among the first notation method and the second notation method, and n+b (b is an odd number greater than or equal to 1 and less than or equal to i) displayed by the same notation method as n.

According to an embodiment, in the method of providing information, when a distance between n+x and n+x+1 (x is an integer greater than or equal to 0 and less than or equal to i-1) belonging to the couplers is defined as D1, a distance between n+x belonging to the couplers and n+x+1 belonging to the intruders is defined as D2, and a distance between n+x+1 belonging to the intruders and n+x+1 belonging to the couplers is defined as D3, conditions D1≥D2 and D1≥D3 are satisfied.

According to another embodiment, the first notation method and the second notation method may be different from each other in one or more selected from a group including a font color of the displayed number, a shade color of the displayed number, and a shade shape of the displayed number.

According to another embodiment, the first notation method and the second notation method may be different from each other in the shade colors of the displayed numbers.

According to another embodiment, the first notation method and the second notation method may be distinguished from each other by the numbers being displayed differently in black and white, respectively, as the shade colors of the numbers

According to another embodiment, the D1 may be greater than the D2 and the D3.

According to another embodiment, the i may be an integer of 2 to 49.

According to another embodiment, the i may be an integer of 9 to 29.

According to another embodiment, when connecting the numbers belonging to the couplers in the order from the smallest to the biggest using line segments, none of the line segments intersect each other.

According to another aspect of the disclosure, there is provided a method for providing a cognitive function test, implemented on a computer, which includes: randomly displaying numbers n to n+i (n is an integer greater than or equal to 0 and i is an integer greater than or equal to 3) on a screen, wherein n is displayed once, n+1 to n+i are displayed twice, respectively, and two identical numbers displayed twice are displayed by a first notation method and a second notation method, which are distinct from each other, respectively, wherein the numbers are classified into two groups of couplers and intruders, wherein the couplers include n defined as a starting point and the n is displayed by the first notation method or the second notation method, wherein the couplers include n+b (b is an odd number greater than or equal to 1 and less than or equal to i) displayed by a notation method different from n, among the first notation method and the second notation method, and n+a (a is an even number greater than or equal to 2 and less than or equal to i) displayed by the same notation method as n, and wherein the intruders include n+a (a is an even number greater than or equal to 2 and less than or equal to i) displayed by a notation method different from n, among the first notation method and the second notation method, and n+b (b is an odd number greater than or equal to 1 and less than or equal to i) displayed by the same notation method as n; receiving information about numbers clicked or sequentially connected by a user; and determining whether the numbers belonging to couplers are sequentially clicked or sequentially connected from the smallest to the biggest, based on the information received from the user, and displaying a result thereof on the screen.

According to an embodiment, in the test providing method, when a distance between n+x and n+x+1 (x is an integer greater than or equal to 0 and less than or equal to i-1) belonging to the couplers is defined as D1, a distance between n+x belonging to the couplers and n+x+1 belonging to the intruders is defined as D2, and a distance between n+x+1 belonging to the intruders and n+x+1 belonging to the couplers is defined as D3, conditions D1≥D2 and D1≥D3 are satisfied.

According to another embodiment, the first notation method and the second notation method may be different from each other in one or more selected from a group including a font color of the displayed number, a shade color of the displayed number, and a shade shape of the displayed number.

According to another embodiment, the i is 2 to 49.

According to another embodiment, when connecting the numbers belonging to the couplers in the order from the smallest to the biggest using line segments, none of the line segments intersect each other.

According to another aspect of the disclosure, there is provided a device for providing a cognitive function test, which includes: a display configured to randomly display numbers n to n+i (n is an integer greater than or equal to 0 and i is an integer greater than or equal to 3) on a screen; an input/output unit configured to receive information about numbers clicked or sequentially connected by a user; and a control unit configured to determine whether the numbers belonging to couplers are sequentially clicked or sequentially connected from the smallest to the biggest, based on the information received from the user, wherein the display is configured to display a determination result of the control unit on the screen, wherein n is displayed once, n+1 to n+i are displayed twice, respectively, and two identical numbers displayed twice are displayed by a first notation method and a second notation method, which are distinct from each other, respectively, wherein the numbers are classified into two groups of couplers and intruders, wherein the couplers include n defined as a starting point and the n is displayed by the first notation method or the second notation method, wherein the couplers include n+b (b is an odd number greater than or equal to 1 and less than or equal to i) displayed by a notation method different from n, among the first notation method and the second notation method, and n+a (a is an even number greater than or equal to 2 and less than or equal to i) displayed by the same notation method as n, and wherein the intruders include n+a (a is an even number greater than or equal to 2 and less than or equal to i) displayed by a notation method different from n, among the first notation method and the second notation method, and n+b (b is an odd number greater than or equal to 1 and less than or equal to i) displayed by the same notation method as n.

The term "unit" as used herein indicates a software or hardware component such as an FPGA or an ASIC, and the "unit" performs a certain function. However, the "unit" is not limited to software or hardware. The "unit" may be configured to reside in an addressable storage medium or may be configured to execute one or more processors. Thus, for example, the "unit" includes components such as software components, object-oriented software components, class components, and task components, processes, functions, properties, procedures, subroutines, segments of program code, drivers, firmware, microcode, circuits, data, databases, data structures, tables, arrays, and variables. The components and the functions provided from the "units" may be combined into a smaller number of components and "units", or may be further divided into additional components and "units".

According to an embodiment, in the test providing device, when a distance between n+x and n+x+1 (x is an integer greater than or equal to 0 and less than or equal to i-1) belonging to the couplers is defined as D1, a distance between n+x belonging to the couplers and n+x+1 belonging to the intruders is defined as D2, and a distance between n+x+1 belonging to the intruders and n+x+1 belonging to the couplers is defined as D3, conditions D1≥D2 and D1≥D3 are satisfied.

According to another embodiment, the first notation method and the second notation method may be different from each other in one or more selected from a group including a font color of the displayed number, a shade color of the displayed number, and a shade shape of the displayed number.

According to another embodiment, the i is 2 to 49.

According to another embodiment, when connecting the numbers belonging to the couplers in the order from the smallest to the biggest using line segments, none of the line segments intersect each other.

According to another aspect of the disclosure, there is provided a computer-readable recording medium including instructions to control a computer system to provide a cognitive function test providing method, and the instructions include: randomly displaying numbers n to n+i (n is an integer greater than or equal to 0 and i is an integer greater than or equal to 3) on a screen, wherein n is displayed once, n+1 to n+i are displayed twice, respectively, and two identical numbers displayed twice are displayed by a first notation method and a second notation method, which are distinct from each other, respectively, wherein the numbers are classified into two groups of couplers and intruders, wherein the couplers include n defined as a starting point and the n is displayed by the first notation method or the second notation method, wherein the couplers include n+b (b is an odd number greater than or equal to 1 and less than or equal to i) displayed by a notation method different from n, among the first notation method and the second notation method, and n+a (a is an even number greater than or equal to 2 and less than or equal to i) displayed by the same notation method as n, and wherein the intruders include n+a (a is an even number greater than or equal to 2 and less than or equal to i) displayed by a notation method different from n, among the first notation method and the second notation method, and n+b (b is an odd number greater than or equal to 1 and less than or equal to i) displayed by the same notation method as n; receiving information about numbers clicked or sequentially connected by a user; and determining whether the numbers belonging to couplers are sequentially clicked or sequentially connected from the smallest to the biggest, based on the information received from the user, and displaying a result thereof on the screen.

According to an embodiment, in the computer-readable recording medium, when a distance between n+x and n+x+1 (x is an integer greater than or equal to 0 and less than or equal to i-1) belonging to the couplers is defined as D1, a distance between n+x belonging to the couplers and n+x+1 belonging to the intruders is defined as D2, and a distance between n+x+1 belonging to the intruders and n+x+1 belonging to the couplers is defined as D3, conditions D1≥D2 and D1≥D3 are satisfied.

According to another embodiment, the first notation method and the second notation method may be different from each other in one or more selected from a group including a font color of the displayed number, a shade color of the displayed number, and a shade shape of the displayed number.

According to another embodiment, the i is 2 to 49.

According to another embodiment, when connecting the numbers belonging to the couplers in the order from the smallest to the biggest using line segments, none of the line segments intersect each other.

### Advantageous Effects of Invention

Using the method and test system of the disclosure, a highly effective and reliable cognitive ability test related to frontal lobe activation can be performed, and the test results can be utilized to provide information for the diagnosis of diseases causing cognitive impairment.

### Brief Description of Drawings

FIG. 1 is a drawing illustrating the basic principle of a test according to an embodiment of the disclosure.
FIG. 2 illustrates an example of a test board.
FIG. 3 illustrates another example of a test board.
FIG. 4 illustrates another example of a test board.
FIG. 5 is a block diagram illustrating a testing device according to an embodiment of the disclosure.
FIG. 6 is a flowchart illustrating a test method according to an embodiment of the disclosure.
FIG. 7 illustrates a graph of the time to complete TMT-B&W Parts A and B depending on education levels (A) and ages (B).

### Best Mode for Carrying out the Invention

Hereinafter, preferred embodiments of the disclosure will be described in detail with reference to the attached drawings.

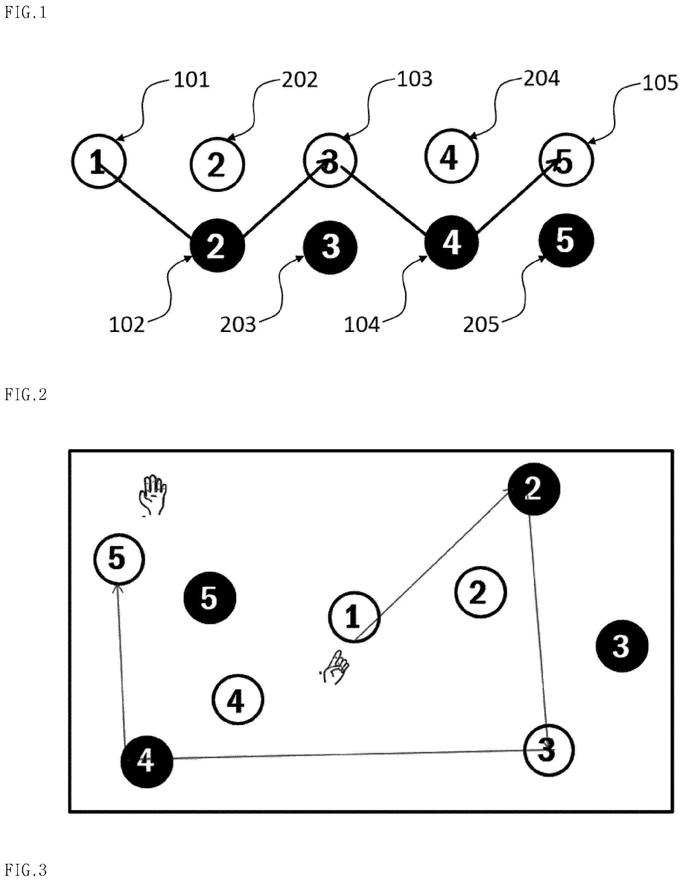

FIG. 1 is a drawing illustrating the basic principle of a test according to an embodiment of the disclosure.

The "test" may be a test for cognitive function related to frontal lobe activation. Referring to FIG. 1, the test according to an example may be performed by providing a user with a cognitive function assessment test board on which a plurality of numbers are arranged, having the user connect the numbers arranged on the test board in order, and determining whether or not the user connects the numbers arranged on the test board in the correct order according to a rule. According to an example, the rule may be to connect the numbers in ascending order from the smallest to the biggest.

The "test board" according to an embodiment of the disclosure may be a piece of paper on which numbers are printed or an electronic device in which numbers are displayed on a screen. In the case of a paper test board, the assessment subject may conduct the test by drawing line segments in order and connecting the numbers selected according to a predetermined rule, or by using fingers or other tools to connect the numbers selected according to a predetermined rule or to point them out in order while the medical staff is visually checking them. In order to facilitate the post-processing of the assessment results, it is desirable to conduct the test by drawing line segments in order and connecting the numbers selected according to a predetermined rule. In the case where the test board is provided as an electronic device including a screen on which numbers are displayed, the same arrangement of numbers may be displayed each time the assessment is conducted, or the arrangement of numbers may vary each time the assessment is conducted. If the arrangement of numbers is the same each time the assessment is conducted, it is easy to compare the test results between different subjects of assessment, and the reliability of the assessment results may be increased. If multiple assessments are conducted on the same subject of assessment, the arrangement of numbers may vary each time the assessment is conducted in order to reduce the bias that may occur due to familiarity with the arrangement of numbers, but the disclosure is not limited thereto.

According to an example, the numbers may be displayed on the test board in a first notation method or a second notation method, which are distinct from each other. In this case, the rule may further include connecting the numbers using the first and second notation methods alternately. That is, the rule may be set to connect the numbers in ascending order from the smallest to the biggest while selecting the notation methods alternately. Referring to the example in FIG. 1, the rule may be to sequentially connect the numbers 101, 102, 103, 104, and 105 according to a predetermined rule.

Referring to FIG. 1, the numbers may be classified into two groups: couplers and intruders. In the example in FIG. 1, couplers 101, 102, 103, 104, and 105 and intruders 202, 203, 204, and 205 may be identified. According to an example, the rule may be to connect only the numbers of couplers, excluding intruders, in ascending order from the smallest to the biggest.

According to an example, numbers n to n+i (n is an integer greater than or equal to 0, and i is an integer greater than or equal to 3) are arranged on the test board such that n is displayed once and such that n+1 to n+i are displayed twice, respectively. One of the two identical numbers displayed twice may be used as a coupler, and the other may be used as an intruder. The coupler and the intruder may be displayed in different ways. For example, the two identical numbers displayed twice may be displayed using the first notation method and the second notation method, respectively, which are distinguished from each other, so that the coupler and the intruder may be visually distinguished.

The first notation method and the second notation method may be different from each other in one or more selected from the group including the font color of the displayed number, the shade color of the displayed number, and the shade shape of the displayed number. In an example, the first notation method and the second notation method may be different in the shade colors of the numbers displayed. In another example, the first notation method and the second notation method may be distinguished from each other by the numbers being displayed differently in black and white, respectively, as the shade colors of the numbers. In the example shown in FIG. 1, the numbers are displayed in black with white shade in the first notation method, and the numbers are displayed in white with black shade in the second notation method.

Referring to FIG. 1, an example in which n=1 and i=4 will be described.

Couplers include n, which is defined as a starting point, and n may be displayed by the first notation method or the second notation method. In the example shown in FIG. 1, the number 1 (101) displayed by the first notation method becomes the starting point.

The couplers may include n+b (b is an odd number greater than or equal to 1 and less than or equal to i) displayed by a notation method different from n, among the first notation method and the second notation method, and n+a (a is an even number greater than or equal to 2 and less than or equal to i) displayed by the same notation method as n, and intruders may include n+a (a is an even number greater than or equal to 2 and less than or equal to i) displayed by a notation method different from n, among the first notation method and the second notation method, and n+b (b is an odd number greater than or equal to 1 and less than or equal to i) displayed by the same notation method as n. In the example shown in FIG. 1, the number 1 is displayed once (101), and the numbers 2 to 5 are displayed twice, respectively, and in the case of the numbers 2 to 5 displayed twice, one is displayed as a coupler 102, 103, 104, or 105 and the other is displayed as an intruder 202, 203, 204, or 205. In the example of FIG. 1, the couplers and the intruders are displayed in different methods.

According to an example, when the distance between n+x and n+x+1 (x is an integer greater than or equal to 0 and less than or equal to i-1) belonging to the couplers is defined as D1, the distance between n+x belonging to the couplers and n+x+1 belonging to the intruders is defined as D2, and the distance between n+x+1 belonging to the intruders and n+x+1 belonging to the couplers is defined as D3, the conditions D1≥D2 and D1≥D3 may be satisfied. According to an example, D1 may be greater than D2 and D3. Accordingly, intruder n+x+1 is located near the line of sight of the user who wishes to connect coupler n+x and coupler n+x+1, thereby effectively hindering the user from correctly connecting the couplers. The example shown in FIG. 1 are illustrated to satisfy the conditions D1≥D2 and D1≥D3, where the distance between the couplers 101 and 102 is D1, the distance between the coupler 101 and the intruder 202 is D2, and the distance between the intruder 202 and the coupler 102 is D3.

Intruders may be used to increase the difficulty of the test. For example, a user may look around coupler n+x in order to move to the next number from coupler n+x, and at this time, the user is more likely to find intruder n+x+1, which is the same number as the next coupler n+x+1 and located closer than the next coupler n+x+1 but displayed in a different notation method therefrom, prior to next coupler n+x+1. Accordingly, the user must make another judgment to exclude intruder n+x+1 in order to correctly move to the next coupler n+x+1. Therefore, the difficulty of the test may be increased due to the intruder.

The above-mentioned i may be 2 to 49. Alternatively, the above-mentioned i may be 9 to 29.

FIG. 2 is an example of a test board. According to an embodiment, the test board in FIG. 2 may be used for a cognitive function test related to frontal lobe activation. FIG. 1 is a drawing for explaining the test rules, whereas FIG. 2 illustrates an actual test board on which numbers randomly arranged. The example in FIG. 2 shows the case where n=1 and i=4.

After providing the test board in FIG. 2 to the user and explaining the rules to the user, a test may be performed by determining whether or not the user sequentially connects the couplers 101, 102, 103, 104, and 105 according to the rules.

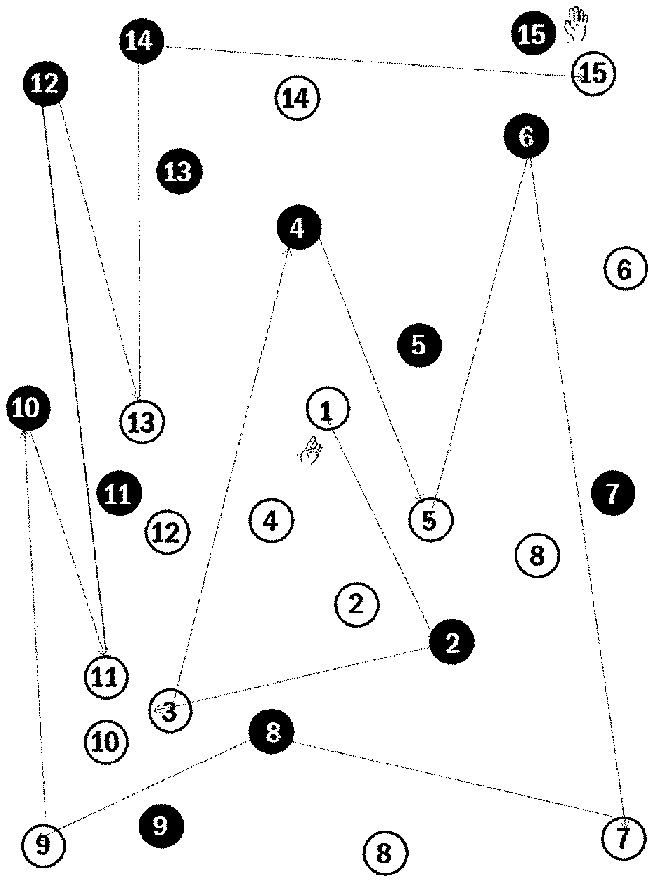

FIG. 3 is another example of a test board. According to an embodiment, the test board in FIG. 3 may be used for a cognitive function test related to frontal lobe activation. The example in FIG. 3 shows the case where n=1 and i=14. In the same manner as described in FIG. 1, the coupler 101 corresponding to the number 1, and the couplers 102 to 115 and intruders 202 to 225 corresponding to the numbers 2 to 15 are displayed on the test board in FIG. 3.

After providing the test board in FIG. 3 to the user and explaining the rules to the user, a test may be performed by determining whether or not the user sequentially connects the couplers 101 to 115 according to the rules.

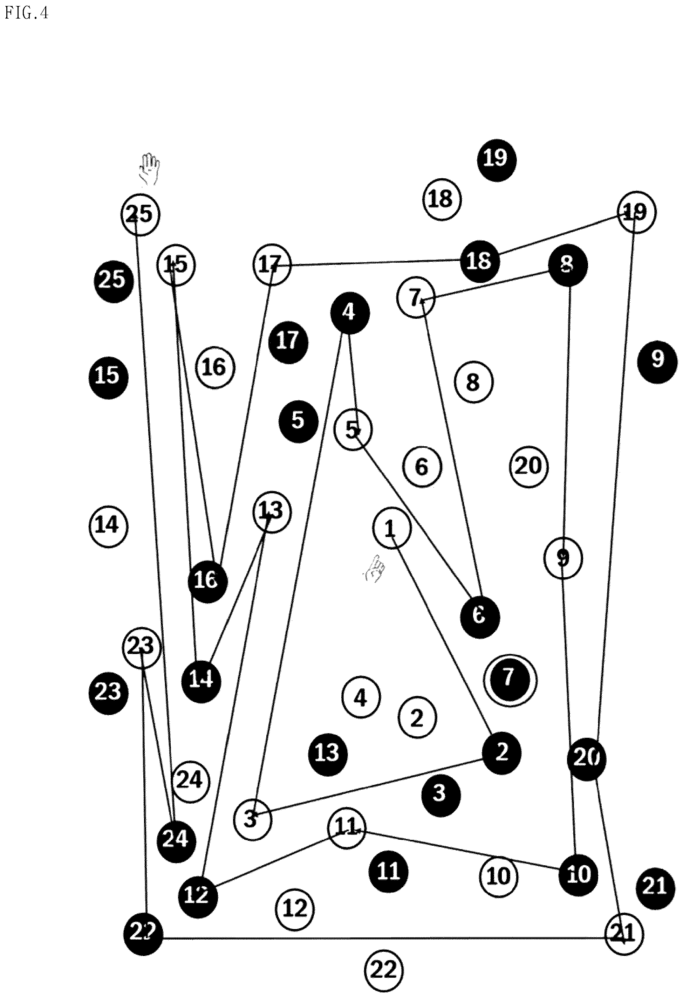

FIG. 4 is another example of a test board. According to an embodiment, the test board in FIG. 4 may be used for a cognitive function test related to frontal lobe activation. The example in FIG. 4 shows the case where n = 1 and i = 24. In the same manner as described in FIG. 1, the coupler 101 corresponding to the number 1, and the couplers 102 to 125 and intruders 202 to 225 corresponding to the numbers 2 to 25 may be displayed on the test board in FIG. 4.

After providing the test board in FIG. 4 to the user and explaining the rules to the user, a test may be performed by determining whether or not the user sequentially connects the couplers 101 to 125 according to the rules.

Referring to FIGS. 2 to 4, a test board according to an example of the disclosure may be configured such that, when connecting the numbers belonging to the couplers sequentially in the order from the smallest to the biggest using line segments, none of the line segments intersect each other.

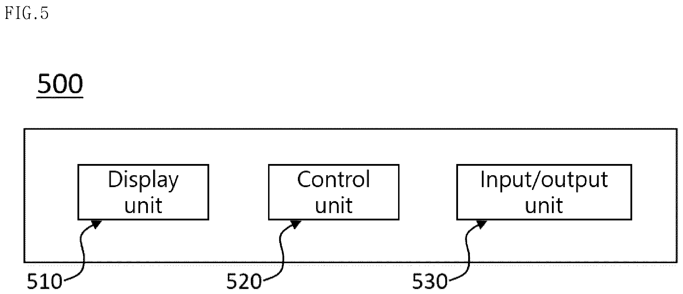

FIG. 5 is a block diagram illustrating a testing device according to an embodiment of the disclosure. A testing device 500 includes a display 510, a control unit 520, and an input/output unit 530.

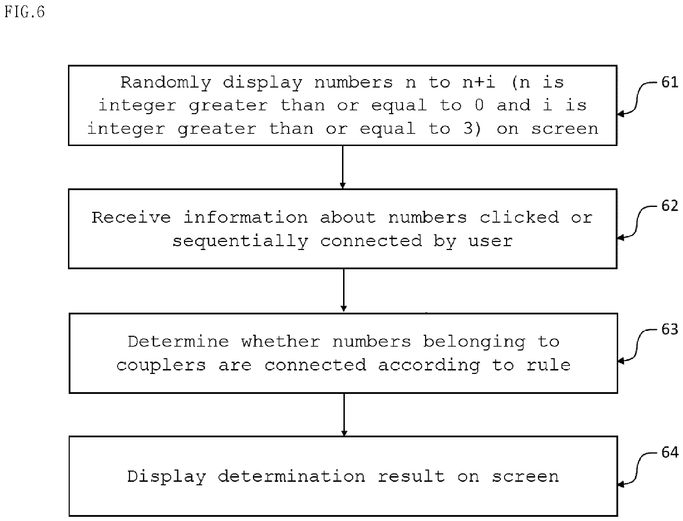

FIG. 6 is a flowchart illustrating a test method according to an embodiment of the disclosure.

Hereinafter, an example of the disclosure will be described with reference to both FIG. 5 and FIG. 6.

In step 61, the display 510 may randomly display numbers n to n+i (n is an integer greater than or equal to 0 and i is an integer greater than or equal to 3) on a screen under the control of the control unit 520.

In step 62, the input/output unit 530 receives information about numbers that the user clicks or sequentially connects.

In step 63, the control unit 520 determines whether or not the numbers belonging to the couplers are connected according to a rule through the information received in step 62. Here, the rule may be to click or connect the numbers belonging to the couplers sequentially from the smallest to the biggest.

In step 64, the display 510 displays the determination result in step 63 on the screen.

Here, n may be displayed once, and n+1 to n+i may be displayed twice, respectively, and the two identical numbers displayed twice may be displayed by the first notation method and the second notation method, respectively, which are distinguished from each other. According to an example, i may be 2 to 49. According to an example, i may be 9 to 29.

The numbers may be classified into two groups, couplers and intruders.

Couplers may include n, which is defined as a starting point. n may be displayed by the first notation method or the second notation method. The couplers may include n+b (b is an odd number greater than or equal to 1 and less than or equal to i) displayed by a notation method different from n, among the first notation method and the second notation method, and n+a (a is an even number greater than or equal to 2 and less than or equal to i) displayed by the same notation method as n. According to an example, when connecting the numbers belonging to the couplers sequentially in the order from the smallest to the biggest using line segments, none of the line segments may intersect each other.

Intruders may include n+a (a is an even number greater than or equal to 2 and less than or equal to i) displayed by a notation method different from n, among the first notation method and the second notation method, and n+b (b is an odd number greater than or equal to 1 and less than or equal to i) displayed by the same notation method as n. When the distance between n+x and n+x+1 (x is an integer greater than or equal to 0 and less than or equal to i-1) belonging to the couplers is defined as D1, the distance between n+x belonging to the couplers and n+x+1 belonging to the intruders is defined as D2, and the distance between n+x+1 belonging to the intruders and n+x+1 belonging to the couplers is defined as D3, the conditions D1≥D2 and D1≥D3 may be satisfied. For example, D1 may be greater than D2 and D3.

The first notation method and the second notation method may be different from each other in one or more selected from the group including the font color of the displayed number, the shade color of the displayed number, and the shade shape of the displayed number. According to an example, the first notation method and the second notation method may be different in the shade colors of the displayed numbers. According to another example, the first notation method and the second notation method may be distinguished from each other by being displayed in one of either black or white, respectively, as the shade colors of the numbers.

The above-described method according to an example of the disclosure may be implemented in the form of a program command executable by a computer means and recorded on a computer-readable medium.

According to an example of the disclosure, a computer program stored on a computer-readable medium may be provided to execute the above-described method.

TMT-B&W, an example of the disclosure, has the advantages of not causing bias due to people with poor literacy or education levels and of enabling cross-cultural comparative studies without bias due to language barriers. TMT-B&W Part B includes intruders from 2 to 25, exhibiting sensitivity to frontal lobe function measurements.

Although the embodiments of the disclosure have been described above with reference to the drawings, those skilled in the art to which the disclosure belongs may understand that various specific modifications may be implemented without changing the technical idea or essential technical features of the disclosure. Therefore, the above-described embodiments are only specific examples of the disclosure, and the disclosure is not limited thereto.

Hereinafter, the disclosure will be described in more detail through experimental examples. The experimental examples are only intended to explain the disclosure in more detail, and it will be clear to those skilled in the art to which the disclosure belongs that the scope of the disclosure presented in the attached claims is not limited to these examples.

### Example 1: Subjects

A health assessment survey (Christensen KJ, Multhaup KS, Nordstrom S, Voss K. A cognitive battery for dementia: Development and measurement characteristics. Psychological Assessment: A Journal of Consulting and Clinical Psychology. 1991;3(2):168.) was conducted for healthy adults who expressed their intention to participate in the study through personal contact in a senior center or the community. A trained psychologist (undergraduate or graduate psychology student) performed the mini-mental status examination and TMT-B&W on the people who were confirmed to have no history of cognitive function impairment. All subjects were informed of the purpose and procedures of the study and gave written consent prior to the study.

### Example 2: TMT-B&W measurement

TMT-B&W was configured by two subsets: TMT-B&W Part A and TMT-B&W Part B. TMT-B&W Part A is configured as 25 circle numbers 1 to 25 with even numbers in black circles and odd numbers in white circles (Figure 1-a). TMT-B&W Part B presents all numbers 2 to 25, excluding number 1, displayed twice. The number 1 is displayed only once in a white circle, and the respective numbers are enclosed in a black circle and a white circle (Figure 1-b).

In TMT-B&W Part A, the subject was instructed to draw a line connecting the numbers in the circles in ascending order. In TMT-B&W Part B, the subject was instructed to connect the numbers in sequential ascending order, alternating between two sets of colors. The time (in seconds) taken to complete the test was measured. A maximum of 300 seconds was allowed to complete each part of the tests.

### Example 3: Statistical Analysis

A correlation analysis was performed to evaluate the correlation between demographic variables. Multiple linear regression analysis was performed to evaluate the relative contributions of age, gender, and education level. A series of analyses of covariance (ANCOVA) were performed using the Bonferroni post-hoc test to identify the primary effects of age and education level. Age was divided into eight categories (19 to 24 years old, 25 to 34 years old, 35 to 44 years old, 45 to 54 years old, 55 to 64 years old, 65 to 74 years old, 75 to 84 years old, and 85 years old or older). Education level was divided into eight categories (illiterate, literate but uneducated group, and 1 to 3 years, 4 to 6 years, 7 to 9 years, 10 to 12 years, 13 to 16 years, and 17 years or more education level) with reference to the Korean education system. Normative data of TMT-B&W were presented as mean, standard deviation, median, and 5th and 95th percentile scores. SPSS version 19.0 was used for all statistical analyses.

### 3-1. Demographic characteristics

Among a total of 1,012 subjects participated in this study, 42% (n=430) were male and 58% (n=582) were female. The demographic findings of the included subjects are shown in Table 1. There was no significant difference in the average age between males and females. The average education level of male subjects was significantly higher than that of female subjects (p<0.001).

**[Table 1]**

| Demographic characteristics of subjects | | | |
|---|---|---|---|
| | Males | Females | Total |
| Number | 430 | 582 | 1012 |
| Age(years), n | 62.8±16.2a | 63.7±16.1 a | 63.3±16.1 a |
| 19-24 | 14b | 26 | 40 |
| 25-34 | 21 | 15 | 36 |
| 35-44 | 19 | 19 | 38 |
| 45-54 | 57 | 90 | 147 |
| 55-64 | 94 | 113 | 207 |
| 65-74 | 106 | 145 | 251 |
| 75-84 | 99 | 147 | 246 |
| 85 or more | 20 | 27 | 47 |
| Education(years), n | 12.2±5.3 a | 9.7±5.9 a | 10.8±5.8 a |
| Illiterate | 8 | 53 | 61 |
| Literate but uneducated group | 9 | 29 | 38 |
| 1-3 | 17 | 37 | 54 |
| 4-6 | 50 | 84 | 134 |
| 7-9 | 50 | 71 | 121 |
| 10-12 | 99 | 135 | 234 |
| 13-16 | 114 | 112 | 226 |
| 17 or more | 83 | 61 | 144 |
| MMSE | 27.4±2.5 a | 26.4±3.6 a | 26.9±3.2 a |

| | | | |
|---|---|---|---|
| ^{a} Mean±SD | | | |

### 3-2. Effects of age, gender, and education level on TMT-B&W

The correlation between demographic variables and scores on TMT-B&W parts A and B showed that age, gender, and education level are significantly correlated with each other, as well as with TMT-B&W parts A and B scores (Table 2).

**[Table 2]**

| Correlation between age, education, gender, and completion time of TMT-B&W parts A and B | | | | |
|---|---|---|---|---|
| | Age | Education | Gender | TMT-B&W Part A |
| Age | | | | |
| Education | -.433* | | | |
| Gender | -.030 | .211* | | |
| TMT-B&W Part A | .554* | -.659* | -.182* | |
| TMT-B&W Part B | .708* | -.704* | -.152* | .751* |

| | | | | |
|---|---|---|---|---|
| * p<0.001 Therefore, multiple regression analysis was performed to identify the independent effect of each variable on TMT-B&W. All of ages, genders, and education levels had a significant effect on TMT-B&W parts A and B scores. In the case of TMT-B&W part A, education level accounted for a more proportion of score variance than age and gender. In TMT-B&W Part B, age, education level, and gender had a significant effect in that order. The results of multiple regression analysis are shown in Table 3. The above three variables showed 53% explanatory power for TMT-B&W Part A and 70% explanatory power for TMT-B&W Part B. Therefore, when interpreting the TMT-B&W results, education level, age, and gender should be considered, and appropriate norms stratified by these variables should be established. | | | | |

**[Table 3]**

| Multiple linear regression analysis of age, education, and gender for TMT-B&W Parts A and B | | | | | |
|---|---|---|---|---|---|
| Variables | *B* | *SE (B)* | *β* | Sig | Partial R² |
| TMT-B&W Part A | | | | | |
| Age | 1.31 | 0.09 | 0.34 | P<0.001 | 0.162 |
| Education | -5.43 | 0.27 | -0.50 | P<0.001 | 0.291 |
| Gender | -8.48 | 2.82 | -0.07 | P<0.01 | 0.008 |
| TMT-B&W Part B | | | | | |
| Age | 2.58 | 0.10 | 0.50 | P<0.001 | 0.399 |
| Education | -6.91 | 0.28 | -0.48 | P<0.001 | 0.371 |
| Gender | -6.05 | 2.99 | -0.036 | P<0.05 | 0.004 |

Regarding the age category, the age was initially subdivided into eight categories (19 to 24 years old, 25 to 34 years old, 35 to 44 years old, 45 to 54 years old, 55 to 64 years old, 65 to 74 years old, 75 to 84 years old, and 85 years old or older). ANCOVA was performed to examine the effect of age, and *post-hoc* analysis was performed to compare the effect of education group among eight age categories. For both TMT-B&W Parts A and B, there were no significant differences between the age categories of 19 to 24 years old, 25 to 34 years old, and 34 to 44 years old and the age groups of 75 to 84 years old and 85 years old or older. Therefore, the age categories were reclassified into five categories (44 years old or younger, 45 to 54 years old, 55 to 64 years old, 65 to 74 years old, and 75 years old or older).

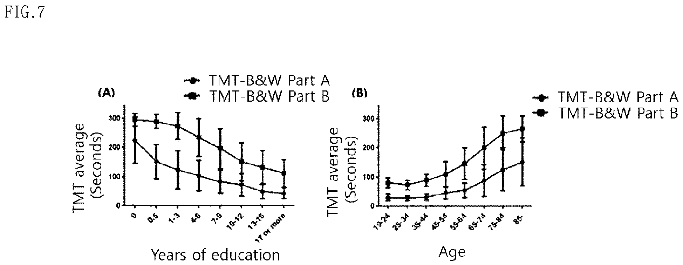

Regarding education level, *post-hoc* analysis among the first eight education levels showed that there were significant differences between any two education level categories, excluding the categories of 13 to 16 years and 17 years or more. Thus, the education level categories were reclassified into seven categories (illiterate, literate but uneducated group, and education of 1 to 3 years, 4 to 6 years, 7 to 9 years, 10 to 12 years, and 13 years or more). FIG. 7 shows the results of ANCOVA, which shows that the time to completion of both TMT-B&W Parts A and B was correlated with age and education level.

### 3. Normative Data

Based on the analysis of the influence of demographic variables, the normative data for TMT-B&W Parts A and B were stratified by age and education level. The normative data for TMT-B&W Parts A and B for males and females are presented in Tables 4 and 5 (TMT-B&W Part A) and Tables 6 and 7 (TMT-B&W Part B).

**[Table 4]**

| Normative data of males for TMT-B&W Part A: mean, standard deviation, median, and scores from the 5th to 95th percentiles | | | | | | | | |
|---|---|---|---|---|---|---|---|---|
| Educati on (years) | | Illite rate | Litera te but uneduc ated group | 1-3 | 4-6 | 7-9 | 10-12 | >13 |
| Age (years) | | | | | | | | |
| 19-44 | N | | | | | | 13 | 41 |
| | Mean | | | | | | 28.69 | 28.98 |
| | SD | | | | | | 7.96 | 9.49 |
| | 5^{th} %ile | | | | | | 16.00 | 14.30 |
| | median | | | | | | 28.00 | 29.00 |
| | 95^{th} %ile | | | | | | 40.40 | 46.90 |
| 45-54 | N | | | | | | 22 | 35 |
| | Mean | | | | | | 46.73 | 36.34 |
| | SD | | | | | | 13.94 | 10.02 |
| | 5^{th} %ile | | | | | | 21.75 | 17.60 |
| | median | | | | | | 43.00 | 38.00 |
| | 95^{th} %ile | | | | | | 81.60 | 51.60 |
| 55-64 | N | | | 3 | 12 | 17 | 21 | 41 |
| | Mean | | | 48.67 | 56.50 | 63.00 | 54.52 | 43.29 |
| | SD | | | 7.02 | 15.49 | 25.65 | 21.50 | 9.12 |
| | 5^{th} %ile | | | 42.00 | 33.00 | 36.00 | 28.40 | 33.00 |
| | median | | | 48.00 | 59.00 | 55.00 | 53.00 | 43.00 |
| | 95^{th} %ile | | | . | 78.9 | 109.0 | 118.60 | 64.80 |
| 65-74 | N | . | 2 | 7 | 15 | 18 | 21 | 43 |
| | Mean | | 155.50 | 85.29 | 80.27 | 86.50 | 65.38 | 54.60 |
| | SD | | 64.35 | 17.21 | 26.66 | 22.81 | 24.92 | 15.86 |
| | 5^{th} %ile | | 110.00 | 64.00 | 48.00 | 47.50 | 34.40 | 30.60 |
| | median | | 155.50 | 87.00 | 72.00 | 81.00 | 57.00 | 51.00 |
| | 95^{th} %ile | | . | . | 125.20 | 128.20 | 121.00 | 90.60 |
| 75-90 | N | 8 | 7 | 7 | 23 | 15 | 22 | 37 |
| | Mean | 220.63 | 132.57 | 109.00 | 130.91 | 106.53 | 91.50 | 71.16 |
| | SD | 76.80 | 51.40 | 43.03 | 51.25 | 36.02 | 27.47 | 21.70 |
| | 5^{th} %ile | 108.00 | 76.00 | 73.00 | 53.20 | 61.00 | 45.15 | 39.80 |
| | median | 247.00 | 118.00 | 104.00 | 115.00 | 100.00 | 93.00 | 69.00 |
| | 95^{th} %ile | . | . | . | 230.20 | 173.50 | 136.35 | 112.80 |

| | | | | | | | | |
|---|---|---|---|---|---|---|---|---|
| N, number; SD, Standard deviation | | | | | | | | |

**[Table 5]**

| Normative data of females for TMT-B&W Part A: mean, standard deviation, median, and scores from the 5th to 95th percentiles | | | | | | | | |
|---|---|---|---|---|---|---|---|---|
| Educat ion (years ) | | Illiter ate | Literat e but uneducated group | 1-3 | 4-6 | 7-9 | 10-12 | >13 |
| Age (years ) | | | | | | | | |
| 19-44 | N | | | | | | 20 | 40 |
| | Mean | | | | | | 34.80 | 27.15 |
| | SD | | | | | | 15.73 | 7.36 |
| | 5th %ile | | | | | | 17.05 | 17.00 |
| | median | | | | | | 31.00 | 25.00 |
| | 95th %ile | | | | | | 83.80 | 39.95 |
| 45-54 | N | | | | 4 | 12 | 30 | 44 |
| | Mean | | | | 81.50 | 53.67 | 48.47 | 39.89 |
| | SD | | | | 73.49 | 24.13 | 18.94 | 13.26 |
| | 5th %ile | | | | 27.00 | 26.00 | 28.30 | 23.50 |
| | median | | | | 54.50 | 46.00 | 43.00 | 36.00 |
| | 95th %ile | | | | . | 91.95 | 108.45 | 73.00 |
| 55-64 | N | 4 | 0 | 4 | 19 | 20 | 37 | 29 |
| | Mean | 72.75 | | 100.0 | 70.26 | 58.85 | 48.97 | 47.03 |
| | SD | 24.06 | | 78.39 | 30.59 | 21.98 | 20.18 | 18.91 |
| | 5th %ile | 55.00 | | 54.00 | 42.00 | 37.15 | 23.70 | 26.00 |
| | median | 64.50 | | 64.50 | 59.00 | 53.00 | 44.00 | 44.00 |
| | 95th %ile | . | | . | 112.50 | 134.30 | 101.50 | 101.00 |
| 65-74 | N | 17 | 9 | 11 | 32 | 20 | 24 | 32 |
| | Mean | 211.2 | 127.89 | 116.18 | 106.06 | 87.25 | 69.25 | 55.25 |
| | SD | 77.40 | 57.21 | 45.78 | 38.58 | 35.37 | 38.48 | 38.44 |
| | 5th %ile | 98.00 | 78.00 | 73.00 | 60.30 | 44.20 | 26.25 | 27.95 |
| | median | 208.00 | 101.00 | 95.00 | 106.00 | 76.00 | 63.00 | 47.00 |
| | 95th %ile | 300.00 | 224.20 | 187.50 | 186.70 | 168.95 | 161.25 | 157.20 |
| 75-90 | N | 32 | 20 | 22 | 29 | 19 | 24 | 28 |
| | Mean | 253.91 | 169.65 | 157.68 | 130.76 | 114.21 | 110.75 | 67.29 |
| | SD | 58.84 | 60.94 | 75.23 | 63.53 | 52.36 | 69.38 | 26.05 |
| | 5th %ile | 146.30 | 91.40 | 76.15 | 57.80 | 53.50 | 42.00 | 24.50 |
| | median | 300.00 | 152.50 | 132.00 | 111.00 | 103.00 | 85.00 | 60.00 |
| | 95th %ile | 300.00 | 298.50 | 300.00 | 278.50 | 276.70 | 275.50 | 119.30 |

| | | | | | | | | |
|---|---|---|---|---|---|---|---|---|
| N, number; SD, Standard deviation | | | | | | | | |

**[Table 6]**

| Normative data of males for TMT-B&W Part B: mean, standard deviation, median, and scores from the 5th to 95th percentiles | | | | | | | | |
|---|---|---|---|---|---|---|---|---|
| Educati on (years) | | Illite rate | Literat e but uneduca ted group | 1-3 | 4-6 | 7-9 | 10-12 | >13 |
| Age (years) | | | | | | | | |
| 19-44 | N | | | | | | 13 | 41 |
| | Mean | | | | | | 81.31 | 77.66 |
| | SD | | | | | | 18.84 | 20.55 |
| | 5th %ile | | | | | | 56.00 | 44.70 |
| | median | | | | | | 77.00 | 75.00 |
| | 95th %ile | | | | | | 117.20 | 115.90 |
| 45-54 | N | | | | | | 22 | 35 |
| | Mean | | | | | | 110.09 | 101.20 |
| | SD | | | | | | 22.68 | 37.76 |
| | 5th %ile | | | | | | 85.15 | 67.40 |
| | median | | | | | | 105.50 | 93.00 |
| | 95th %ile | | | | | | 172.80 | 189.40 |
| 55-64 | N | | | 3 | 12 | 17 | 21 | 41 |
| | Mean | | | 175.67 | 150.75 | 150.35 | 143.00 | 116.78 |
| | SD | | | 4.50 | 34.96 | 40.21 | 44.01 | 27.98 |
| | 5th %ile | | | 171.00 | 100.00 | 95.00 | 82.40 | 73.00 |
| | median | | | 176.00 | 143.00 | 140.00 | 144.00 | 114.00 |
| | 95th %ile | | | . | 201.80 | 212.40 | 229.80 | 170.40 |
| 65-74 | N | | 2* | 7 | 15 | 18 | 21 | 43 |
| | Mean | . | . | 248.14 | 223.07 | 191.1 | 153.05 | '41.60 |
| | SD | | | 55.29 | 51.40 | 62.25 | 29.07 | 44.42 |
| | 5th %ile | | | 156.00 | 141.00 | 118.00 | 86.80 | 86.20 |
| | median | | | 262.00 | 214.00 | 178.00 | 162.00 | 135.00 |
| | 95th %ile | | | . | 300.00 | 300.00 | 191.40 | 229.80 |
| 75-90 | N | 8* | 7 | 7 | 23 | 15 | 22 | 37 |
| | Mean | | 277.14 | 287.71 | 268.09 | 256.93 | 234.50 | 199.59 |
| | SD | | 34.39 | 27.02 | 44.78 | 46.27 | 52.30 | 59.16 |
| | 5th %ile | | 220.00 | 227.00 | 156.00 | 144.00 | 131.45 | 102.90 |
| | median | | 300.00 | 300.00 | 297.00 | 262.00 | 241.00 | 207.00 |
| | 95th %ile | | . | . | 300.00 | 300.00 | 300.00 | 300.00 |

| | | | | | | | | |
|---|---|---|---|---|---|---|---|---|
| * None of the individuals in this category completed the test within 300 seconds. N, number; SD, Standard deviation | | | | | | | | |

**[Table 7]**

| Normative data of females for TMT-B&W Part B: mean, standard deviation, median, and scores from the 5th to 95th percentiles | | | | | | | | |
|---|---|---|---|---|---|---|---|---|
| Educatio n (years) | | Illite rate | Literate but uneducat ed group | 1-3 | 4-6 | 7-9 | 10-12 | >13 |
| Age (years) | | | | | | | | |
| 19-44 | N | | | | | | | |
| | | | | | | | 20 | 40 |
| | Mean | | | | | | | |
| | | | | | | | 91.35 | 77.95 |
| | SD | | | | | | | |
| | | | | | | | 19.56 | 17.40 |
| | 5th %ile | | | | | | | |
| | | | | | | | 69.10 | 53.20 |
| | median | | | | | | | |
| | | | | | | | 84.50 | 76.00 |
| | 95th %ile | | | | | | | |
| | | | | | | | 140.0 | 113.6 |
| | | | | | | | 5 | 5 |
| 45-54 | | | | | | 12 | 30 | |
| | | | | | 4 | | | |
| | | | | | | 146.7 | 119.3 | 44 |
| | N | | | | 157.7 | | | |
| | | | | | | 5 | 3 | 94.89 |
| | Mean | | | | 5 | | | |
| | | | | | | 61.62 | 54.82 | 27.99 |
| | SD | | | | 87.64 | | | |
| | | | | | | 79.00 | 63.75 | 61.75 |
| | 5th %ile | | | | 98.00 | | | |
| | | | | | | 146.0 | 105.0 | 86.50 |
| | median | | | | 122.5 | | | |
| | | | | | | 0 | 0 | 155.2 |
| | 95th %ile | | | | 0 | | | |
| | | | | | | 246.6 | 280.3 | 5 |
| | | | | | | 0 | 0 | |
| 55-64 | | | | | 19 | | | |
| | | | | 4 | | 20 | 37 | 29 |
| | | | | | 198.4 | | | |
| | | | | 227.5 | | 169.1 | 135.2 | 120.9 |
| | N | 4 | 0 | | 7 | | | |
| | | | | 0 | | 0 | 2 | 0 |
| | Mean | 239.5 | | | 63.96 | | | |
| | | | | 84.61 | | 63.53 | 41.11 | 46.05 |
| | SD | 68.36 | | | 122.0 | | | |
| | | | | 140.0 | | 88.85 | 81.60 | 72.00 |
| | 5th %ile | 154.00 | | | 0 | | | |
| | | | | 0 | | 153.0 | 128.0 | 108.0 |
| | median | 252.00 | | | 172.0 | | | |
| | | | | 235.0 | | 0 | 0 | 0 |
| | 95th %ile | | | | 0 | | | |
| | | | | 0 | | 300.0 | 229.7 | 224.6 |
| | | | | | 299.1 | | | |
| | | | | | | 0 | 0 | 0 |
| | | | | | 0 | | | |
| 65-74 | N | 17 | 9 | | | | | |
| | | | | 11 | 32 | 20 | 24 | 32 |
| | Mean | 299.82 | 291.11 | | | | | |
| | SD | 0.73 | 18.33 | 285.8 | 243.6 | 208.6 | 180.2 | 145.3 |
| | 5th %ile | 297.00 | 250.00 | 2 | 3 | 5 | 5 | 8 |
| | median | 300.00 | 300.00 | 17.28 | 64.02 | 70.18 | 65.80 | 42.63 |
| | 95th %ile | 300.00 | 300.00 | 220.0 | 119.0 | 104.5 | 93.50 | 84.05 |
| | | | | 0 | 5 | 5 | 162.5 | 135.0 |
| | | | | 297.0 | 266.5 | 218.5 | 0 | 0 |
| | | | | 0 | 0 | 0 | 300.0 | 235.4 |
| | | | | 300.0 | 300.0 | 300.0 | 0 | 0 |
| | | | | 0 | 0 | 0 | | |
| 75-90 | | | | 22 | 29 | 19 | 24 | |
| | | | | | | | | 28 |
| | | | | 295.7 | 276.5 | 240.9 | 237.3 | |
| | | | | | | | | 187.3 |
| | N | 32* | 20 | 3 | 9 | 5 | 8 | |
| | | | | | | | | 6 |
| | Mean | | 295.85 | 20.04 | 37.26 | 57.97 | 46.68 | |
| | | | | | | | | 50.98 |
| | SD | | 15.47 | 220.1 | 181.0 | 118.0 | 163.2 | |
| | | | | | | | | 84.60 |
| | 5th %ile | | 233.90 | 0 | 0 | 0 | 5 | |
| | | | | | | | | 202.0 |
| | median | | 300.00 | 300.0 | 300.0 | 232.0 | 233.5 | |
| | | | | | | | | 0 |
| | 95th %ile | | 300.00 | 0 | 0 | 0 | 0 | |
| | | | | | | | | 275.7 |
| | | | | 300.0 | 300.0 | 300.0 | 300.0 | |
| | | | | | | | | 0 |
| | | | | 0 | 0 | 0 | 0 | |

| | | | | | | | | |
|---|---|---|---|---|---|---|---|---|
| * None of the individuals in this category completed the test within 300 seconds. N, number; SD, Standard deviation | | | | | | | | |

### [Described of Reference numerals]

101, 102, 103, 104, 105: Coupler
202, 203, 204, 205: Intruder
500: Testing device
510: Display
520: Control unit
530: Input/output unit

## Claims

1. A cognitive function assessment test board on which numbers n to n+i (n is an integer greater than or equal to 0 and i is an integer greater than or equal to 3) are arranged, wherein n is displayed once, n+1 to n+i are displayed twice, respectively, and two identical numbers displayed twice are displayed by a first notation method and a second notation method, which are distinct from each other, respectively,
wherein the numbers are classified into two groups of couplers and intruders,
wherein the couplers comprise n defined as a starting point and the n is displayed by the first notation method or the second notation method,
wherein the couplers comprise n+b (b is an odd number greater than or equal to 1 and less than or equal to i) displayed by a notation method different from n, among the first notation method and the second notation method, and n+a (a is an even number greater than or equal to 2 and less than or equal to i) displayed by the same notation method as n, and
wherein the intruders comprise n+a (a is an even number greater than or equal to 2 and less than or equal to i) displayed by a notation method different from n, among the first notation method and the second notation method, and n+b (b is an odd number greater than or equal to 1 and less than or equal to i) displayed by the same notation method as n.

2. The cognitive function assessment test board according to claim 1,
wherein, when a distance between n+x and n+x+1 (x is an integer greater than or equal to 0 and less than or equal to i-1) belonging to the couplers is defined as D1, a distance between n+x belonging to the couplers and n+x+1 belonging to the intruders is defined as D2, and a distance between n+x+1 belonging to the intruders and n+x+1 belonging to the couplers is defined as D3, conditions D1≥D2 and D1≥D3 are satisfied.

3. The cognitive function assessment test board according to claim 1,
wherein the first notation method and the second notation method are different from each other in one or more selected from a group including a font color of the displayed number, a shade color of the displayed number, and a shade shape of the displayed number.

4. The cognitive function assessment test board according to claim 3,
wherein the first notation method and the second notation method are different from each other in the shade colors of the displayed numbers.

5. The cognitive function assessment test board according to claim 4,
wherein the first notation method and the second notation method are distinguished from each other by the numbers being displayed differently in black and white, respectively, as the shade colors of the numbers.

6. The cognitive function assessment test board according to claim 1,
wherein the D1 is greater than the D2 and the D3.

7. The cognitive function assessment test board according to claim 1,
wherein the i is 2 to 49.

8. The cognitive function assessment test board according to claim 7,
wherein the i is 9 to 29.

9. The cognitive function assessment test board according to claim 1,
wherein, when connecting the numbers belonging to the couplers in the order from the smallest to the biggest using line segments, none of the line segments intersect each other.

10. A method of providing information for cognitive function assessment, the method comprising:
randomly displaying numbers n to n+i (n is an integer greater than or equal to 0 and i is an integer greater than or equal to 3) on a screen;
receiving information about numbers clicked or sequentially connected by a user; and
determining whether the numbers belonging to couplers are sequentially clicked or sequentially connected from the smallest to the biggest, based on the information received from the user, and displaying a result thereof on the screen,
wherein n is displayed once, n+1 to n+i are displayed twice, respectively, and two identical numbers displayed twice are displayed by a first notation method and a second notation method, which are distinct from each other, respectively, wherein the numbers are classified into two groups of couplers and intruders, wherein the couplers comprise n defined as a starting point and the n is displayed by the first notation method or the second notation method, wherein the couplers comprise n+b (b is an odd number greater than or equal to 1 and less than or equal to i) displayed by a notation method different from n, among the first notation method and the second notation method, and n+a (a is an even number greater than or equal to 2 and less than or equal to i) displayed by the same notation method as n, and wherein the intruders comprise n+a (a is an even number greater than or equal to 2 and less than or equal to i) displayed by a notation method different from n, among the first notation method and the second notation method, and n+b (b is an odd number greater than or equal to 1 and less than or equal to i) displayed by the same notation method as n.

11. The method of providing information for cognitive function assessment according to claim 10,
wherein, when a distance between n+x and n+x+1 (x is an integer greater than or equal to 0 and less than or equal to i-1) belonging to the couplers is defined as D1, a distance between n+x belonging to the couplers and n+x+1 belonging to the intruders is defined as D2, and a distance between n+x+1 belonging to the intruders and n+x+1 belonging to the couplers is defined as D3, conditions D1≥D2 and D1≥D3 are satisfied.

12. The method of providing information for cognitive function assessment according to claim 11,
wherein the first notation method and the second notation method are different from each other in one or more selected from a group including a font color of the displayed number, a shade color of the displayed number, and a shade shape of the displayed number.

13. The method of providing information for cognitive function assessment according to claim 12,
wherein the first notation method and the second notation method are different from each other in the shade colors of the displayed numbers.

14. The method of providing information for cognitive function assessment according to claim 13,
wherein the first notation method and the second notation method are distinguished from each other by the numbers being displayed differently in black and white, respectively, as the shade colors of the numbers

15. The method of providing information for cognitive function assessment according to claim 10,
wherein the D1 is greater than the D2 and the D3.

16. The method of providing information for cognitive function assessment according to claim 10,
wherein the i is 2 to 49.

17. The method of providing information for cognitive function assessment according to claim 16,
wherein the i is 9 to 29.

18. The method of providing information for cognitive function assessment according to claim 10,
wherein, when connecting the numbers belonging to the couplers in the order from the smallest to the biggest using line segments, none of the line segments intersect each other.

19. A device for providing a cognitive function test, the device comprising:
a display configured to randomly display numbers n to n+i (n is an integer greater than or equal to 0 and i is an integer greater than or equal to 3) on a screen;
an input/output unit configured to receive information about numbers clicked or sequentially connected by a user; and
a control unit configured to determine whether the numbers belonging to couplers are sequentially clicked or sequentially connected from the smallest to the biggest, based on the information received from the user,
wherein the display is configured to display a determination result of the control unit on the screen,
wherein n is displayed once, n+1 to n+i are displayed twice, respectively, and two identical numbers displayed twice are displayed by a first notation method and a second notation method, which are distinct from each other, respectively, wherein the numbers are classified into two groups of couplers and intruders, wherein the couplers comprise n defined as a starting point and the n is displayed by the first notation method or the second notation method, wherein the couplers comprise n+b (b is an odd number greater than or equal to 1 and less than or equal to i) displayed by a notation method different from n, among the first notation method and the second notation method, and n+a (a is an even number greater than or equal to 2 and less than or equal to i) displayed by the same notation method as n, and wherein the intruders comprise n+a (a is an even number greater than or equal to 2 and less than or equal to i) displayed by a notation method different from n, among the first notation method and the second notation method, and n+b (b is an odd number greater than or equal to 1 and less than or equal to i) displayed by the same notation method as n.

20. The device for providing a cognitive function test according to claim 19,
wherein, when a distance between n+x and n+x+1 (x is an integer greater than or equal to 0 and less than or equal to i-1) belonging to the couplers is defined as D1, a distance between n+x belonging to the couplers and n+x+1 belonging to the intruders is defined as D2, and a distance between n+x+1 belonging to the intruders and n+x+1 belonging to the couplers is defined as D3, conditions D1≥D2 and D1≥D3 are satisfied.

21. The device for providing a cognitive function test according to claim 20,
wherein the first notation method and the second notation method are different from each other in one or more selected from a group including a font color of the displayed number, a shade color of the displayed number, and a shade shape of the displayed number.

22. The device for providing a cognitive function test according to claim 20,
wherein the i is 2 to 49.

23. The device for providing a cognitive function test according to claim 20,
wherein, when connecting the numbers belonging to the couplers in the order from the smallest to the biggest using line segments, none of the line segments intersect each other.

24. A computer program stored in a computer-readable recording medium for executing, on a computer, respective operations according to any one of claims 10 to 18.
